# EUROPÄISCHE PATENTSCHRIFT

(11) **EP 2 842 596 B1**
(45) Veröffentlichungstag und Bekanntmachung des Hinweises auf die Patenterteilung: **07.03.2018**
(21) Anmeldenummer: 14002731.9
(22) Anmeldetag: 05.08.2014
(51) Int. Cl.: F16K 7/00, A61M 39/28, F16L 55/10

(54) **Schlauchklemme zum Halten und Verschließen eines Schlauchs**
Hose clamp for holding and closing a hose
Bride de serrage pour tuyaux destinée à maintenir et fermer un tuyau

(30) Priorität: 26.08.2013 DE 102013014217
(43) Veröffentlichungstag der Anmeldung: 04.03.2015
(73) Patentinhaber: Thomas Magnete GmbH, 57562 Herdorf (DE)
(72) Erfinder: Hamm, Wolfgang, 57520 Mauden (DE); Sauer, Daniel, 57074 Siegen (DE)

(56) Entgegenhaltungen:
- EP-A1- 2 583 716
- WO-A2-2010/105121
- DE-B3- 10 322 007
- FR-A1- 2 908 176

## Beschreibung

Die Erfindung betrifft eine Schlauchklemme entsprechend dem Oberbegriff des ersten Patentanspruchs.

### Stand der Technik

Schlauchklemmen zum Verschließen und Öffnen von hochelastischen Schläuchen sind bekannt. Sie klemmen den Schlauch so zusammen, dass der Durchfluss eines Fluids durch den Schlauch verhindert wird, solange die Schlauchklemme aktiviert ist, und lösen die Klemmwirkung wieder, wenn die Schlauchklemme abgenommen oder entriegelt wird.
Bekannte Schlauchklemmen erfordern in vielen Fällen, den Schlauch längs in die Schlauchklemme einzuführen, das setzt vorher ein Lösen des Schlauchs von einem Behälter oder einem Ventil voraus.
Ein zweiter vielfach anzutreffender Mangel betrifft die Verrastung der Schlauchklemme, es gibt dabei in der Regel nur zwei Stellungen, lose bei offenem Schlauch und unverschieblich bei verschlossenem Schlauch; erwünscht ist eine dritte Stellung bei offenem Schlauch und sicher aufliegender Schlauchklemme, die nicht zusätzlich gehalten werden muss.
Wegen der Doppelfunktion der herkömmlichen Verrastung, nämlich Halten der Schlauchklemme und Verschließen des Schlauchs, muss zum Öffnen des Schlauchs immer die Verrastung gelöst werden, das erschwert die Handhabung insbesondere beim Einsatz von Schlauchpumpen, denn hier ist es wünschenswert, beim Einlegen des Schlauchs mit der Schlauchklemme in die Schlauchpumpe die Verrastung nicht zu lösen.

Die Druckschrift WO 2010/105121 A2 zeigt Schlauchklemmen mit einem Rastmechanismus und einem hebelbetätigten Getriebe zum Verschließen und Öffnen des Schlauchs. Die dort gezeigten Schlauchklemmen ermöglichen ein seitliches Einlegen des Schlauches und eine Verschiebung der Schlauchklemme relativ zum Schlauch, solange die Schlauchklemmen den Schlauch noch nicht verschließen. Die Druckschriften FR 2908176 A1, EP 2583716 A1 und DE 10322007 B3 zeigen weitere Schlauchklemmen, die allerdings kein geeignetes Lösegetriebe aufweisen.

Bekannte Schlauchklemmen ermöglichen die folgenden Betätigungen:
- Aufsetzen der Schlauchklemme von der Seite auf den Schlauch
- Verriegeln der Schlauchklemme mit Verschließen des Schlauchs
In einer verbesserten Ausführung erfolgt das Verriegeln in zwei Stufen, in einer ersten Stufe nur zur Sicherung des Aufsetzens ohne Verschließen des Schlauchs und in einer zweiten Stufe zur nicht verschieblichen Festsetzung der Schlauchklemme und zum Verschließen des Schlauchs. Ein Lösen der zweiten Stufe durch eine drückende Betätigung ist dabei nicht möglich.

### Aufgabe

Zusätzlich zu den Betätigungen der bekannten Schlauchklemmen soll die erfindungsgemäße Schlauchklemme ein Öffnen des Schlauchs durch ein drückendes Betätigungsorgan gegen eine Federkraft ohne Lösung der Verriegelung ermöglichen.

### Lösung

Die Aufgaben werden durch die Merkmale des ersten Patentanspruchs gelöst, in Verbindung mit den darauf folgenden Unteransprüchen.
Das seitliche Aufsetzen der Schlauchklemme auf den Schlauch wird ermöglicht, indem die aus einem Gehäuse und einem gelenkig daran befestigten Bügel bestehende Schlauchklemme aufgeklappt wird und der Schlauch seitlich in die offene Schlauchklemme eingelegt wird. Beim Zuklappen des Bügels auf das Gehäuse legt sich der Schlauch in halbrunde Aufnahmen des Gehäuses einerseits und des Bügels andererseits und hält damit die Schlauchklemme auf dem Schlauch.
Der Schlauch muss nicht längs in die Schlauchklemme eingeführt werden und daher auch nicht von seinen Anschlussfestlegungen gelöst werden.
Ein Rastmechanismus hält den Bügel in der zugeklappten Lage, dabei kann auch der Schlauch so weit zusammengedrückt werden, dass er verschlossen wird.

Wenn eine hohe Kraft auf den Schlauch ausgeübt wird, ist die Schlauchklemme in der Regel nicht mehr auf dem Schlauch verschieblich.

Wenn die Schlauchklemme einen zweistufigen Rastmechanismus hat, kann man eine erste Stufe nutzen, um zunächst nur die Schlauchklemme unverlierbar zu halten, aber den Schlauch noch nicht zu schließen und die Schlauchklemme noch verschieblich zu lassen.
Erst nach dem Einrasten der zweiten Stufe wird der Schlauch verschlossen und die Schlauchklemme unverschieblich festgesetzt, indem eine Rippe im Bügel den Schlauch gegen einen Balken im Gehäuse drückt.
Dabei wird dann auch eine Feder gespannt, die den Balken hält, und die Verspannung der Feder gleicht Lagetoleranzen und Unterschiede in den Schlauchabmessungen sowie in der Elastizität des Schlauchs aus. Die Feder begrenzt auch die auf den Schlauch ausgeübte Klemmkraft.
Alternativ oder zusätzlich kann auch die Rippe im Bügel elastisch abgestützt ausgeführt sein.
Der zweistufige Rastmechanismus wird durch Haken an dem Bügel verrastet, die in eine gehäusefeste Traverse eingreifen. Um zwei Stufen der Verrastung darzustellen, haben die Haken zum Beispiel unterschiedliche Längen.
Die Haken können auch gehäusefest sein, wenn die Traverse Teil des Bügels ist. Um den Schlauch öffnen zu können, ohne die Verrastung zu lösen, ist der genannte federgestützte Balken mit einem Taster verbunden. Wird dieser betätigt, öffnet sich der Schlauch, weil der Balken gegen die Kraft der Feder von der Rippe weg bewegt wird. Die Kombination von Feder, Balken und Taster wird Lösegetriebe genannt, es ist über die Feder am Gehäuse befestigt.
Bei der alternativen Ausführung mit federnd abgestütztem Balken im Bügel ist konsequenterweise der Lösemechanismus im Bügel angeordnet. Vorteilhafterweise werden das Gehäuse, der Bügel und das Lösegetriebe aus Kunststoff durch Spritzgießen geformt. Verbindet man den Bügel mit dem Gehäuse stoffschlüssig durch ein Filmscharnier, können alle Bestandteile der Schlauchklemme mit einem Werkzeug hergestellt und zusammen entnommen werden.

Alternativ lässt sich die Schlauchklemme aus zwei Teilen aufbauen, dazu wird der Bügel mit Haken gelenkig mit dem Gehäuse verbunden, wobei das Gehäuse Ausnehmungen aufweist, in die die Haken eingreifen, oder das Gehäuse weist Haken auf, die in Ausnehmungen des Bügels eingreifen.

### Anwendung:

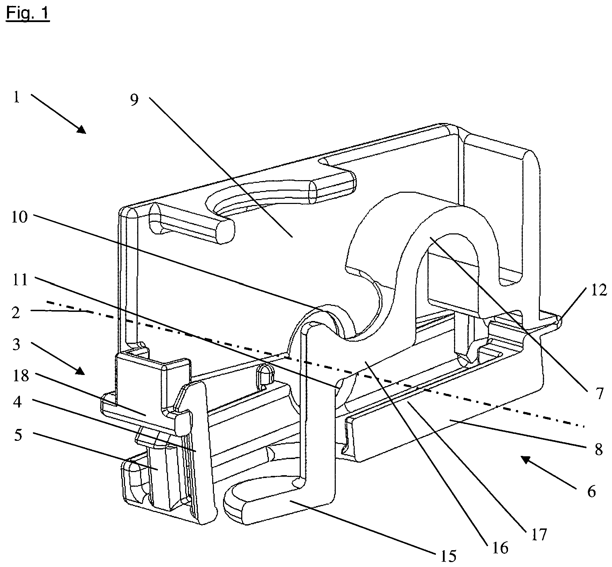

Die Schlauchklemme findet vorrangig in der Medizintechnik Anwendung, zum Beispiel für Schlauchleitungen für die künstliche Ernährung.
Bilder: Fig. 1 zeigt eine erfindungsgemäße Schlauchklemme im mittigen Längsschnitt, verrastet in der ersten Stufe

### Beispielhafte Ausführung

Eine Schlauchklemme (1) gemäß Fig. 1 wird auf einen hochelastischen Schlauch (2) aufgesetzt und von diesem Schlauch gehalten.

Das Aufsetzen der Schlauchklemme auf den Schlauch erfolgt vorzugsweise seitlich durch ein Aufklappen der Schlauchklemme, die aus einem Gehäuse (9) und einem daran gelenkig befestigten Bügel (8) besteht.

Der Bügel (8) ist so weit von dem Gehäuse (9) abklappbar, dass der Schlauch (2) sich in gehäuseseitige halbrunde Aufnahmen (10) und in bügelseitige halbrunde Aufnahmen (11) einlegen lässt.

Die Schlauchklemme weist einen das Gehäuse und den Bügel verbindenden Rastmechanismus (3) auf, und nach dem vollständigen Einrasten des Rastmechanismus wird sie unverschieblich auf dem Schlauch gehalten und der Schlauch wird verschlossen.

Die Schlauchklemme (1) enthält auch ein Lösegetriebe (6), das bei einer Betätigung, ohne den Rastmechanismus (3) zu entrasten, den Schlauch öffnet, solange die Betätigung gegen die Kraft einer Feder (7) anhält.

Der Rastmechanismus (3) ist vorteilhafterweise für eine zweistufige Betätigung eingerichtet, wobei der Rastmechanismus nach einem Aufsetzen der Schlauchklemme (1) auf den Schlauch (2) und seiner Betätigung in einer ersten Stufe die Schlauchklemme verschieblich auf dem Schlauch hält, und ihn noch nicht verschließt.

In einer zweiten Stufe der Betätigung des Rastmechanismus hält dieser die Schlauchklemme auf dem Schlauch unverschieblich und verschließt den Schlauch, indem er ihn zwischen einem Balken (16) und einer Rippe (17) einklemmt.

Dabei wird die den Balken (16) tragende Feder (7) gespannt, sie hält die Kraft zum Einklemmen des Schlauchs in zulässigen Grenzen und übt eine Gegenkraft auf den Rastmechanismus aus.

In der ersten Stufe wird der Rastmechanismus (3) bevorzugt durch einen ersten federnden Haken (4) gehalten und in der zweiten Stufe wird der Rastmechanismus durch einen oder mehrere weitere federnde Haken (5) gehalten, die vorzugsweise kürzer sind als der Haken (4). Durch die unterschiedlichen Längen der Haken (4) und (5) wird erreicht, dass zunächst die erste Stufe und dann die zweite Stufe einrasten.

Die erste und die zweite Stufe der Betätigung des Rastmechanismus (3) werden beide durch einen Druck auf den an dem Gehäuse (9) gelenkig abgestützten Bügel (8) bewirkt, unterschieden nur durch die Kraft oder den Weg der Betätigung.

Der Bügel (8) führt die federnden Haken (4) und (5), die sich in eine Traverse (19) einhaken, die Bestandteil eines Gehäuses (9) ist. In einer alternativen Ausführung sind die Haken gehäusefest und die die Traverse ist Bestandteil des Bügels.

Der aus Kunststoff hergestellte Bügel (8) ist vorzugsweise mittels eines Filmscharniers (12) aus Kunststoff mit dem ebenfalls aus Kunststoff hergestellten Gehäuse (9) verbunden, alternativ dringen Haken des Bügels in Ausnehmungen des Gehäuses ein und stellen eine gelenkige Verbindung zwischen den beiden Bauteilen her, oder Haken des Gehäuses dringen in Ausnehmungen des Bügels ein.

Das Lösegetriebe (6) ist vorzugsweise mit dem Gehäuse (9) verbunden und besteht aus einem Taster (15), dem Balken (16) und der Feder (7). Dabei ist in einem vollständig eingerasteten Zustand des Rastmechanismus (3) und einem nicht betätigten Zustand des Lösegetriebes (6) der Schlauch (2) zwischen einer Rippe (17) des Bügels (8) und dem Balken (16) durch die Kraft der Feder (7) so stark eingeklemmt, dass ein Durchfluss von Fluid durch den Schlauch verhindert wird.

Alternativ ist das Lösegetriebe in dem Bügel (8) angeordnet, dann ist die Rippe (17) am Gehäuse (9) fest.

Ausgehend von dem vollständig eingerasteten Zustand des Rastmechanismus entfernt eine drückende Betätigung des Tasters (15) gegen die Kraft der Feder (7) den Balken (16) so weit von der Rippe (17), dass der Schlauch geöffnet wird, solange die Betätigung des Tasters (15) anhält.

Wird der Taster (15) nicht mehr betätigt, schließt die Feder (7) den Schlauch wieder, indem sie den Balken wieder mit ausreichender Kraft an die Rippe drückt.

Vorzugsweise sind alle Bestandteile der Schlauchklemme aus dem gleichen Kunststoff mit einem gemeinsamen Werkzeug herstellbar und das Werkzeug enthält keinen Schieber. Die Schlauchklemme wird in einem geöffneten Zustand geformt und in diesem Zustand weisen die Bauteile keine Hinterschnitte auf.

### Liste der Bezugszeichen

1. Schlauchklemme
2. Schlauch
3. Rastmechanismus
4. Haken
5. Haken
6. Lösegetriebe
7. Feder
8. Bügel
9. Gehäuse
10. Aufnahme
11. Aufnahme
12. Filmscharnier
15. Taster
16. Balken
17. Rippe
18.Traverse

## Patentansprüche

1. Schlauchklemme (1) zum Aufsetzen auf einen hochelastischen Schlauch (2),
wobei die Schlauchklemme (1) ein Gehäuse (9), einen Bügel (8) und einen die beiden Bauteile verbindenden Rastmechanismus (3) aufweist, der nach dem vollständigen Einrasten die Schlauchklemme unverschieblich auf dem Schlauch hält und auch den Schlauch verschließt,
wobei die Schlauchklemme (1) ein Lösegetriebe (6) enthält, das bei einer Betätigung, unter Aufrechterhaltung der Rastfunktion des Rastmechanismus (3), dem Schlauch eine Rückfederung in eine geöffnete Form ermöglicht, solange die Betätigung des Lösegetriebes anhält,
wobei der Rastmechanismus (3) für eine zweistufige Betätigung eingerichtet ist, und wobei der Rastmechanismus nach einem Aufsetzen der Schlauchklemme (1) auf den Schlauch (2) und seiner Betätigung in einer ersten Stufe die Schlauchklemme verschieblich in Aufnahmen (10) und (11) auf dem Schlauch hält, aber ihn noch nicht verschließt
und wobei der Rastmechanismus nach seiner Betätigung in einer zweiten Stufe sowohl die Schlauchklemme auf dem Schlauch unverschieblich hält als auch den Schlauch verschließt, indem er ihn zwischen einer Rippe (17) und einem Balken (16) einklemmt und dabei eine den Balken abstützende Feder (7) spannt
**dadurch gekennzeichnet, dass** das Lösegetriebe (6) aus einem Taster (15), dem Balken (16) und der Feder (7) besteht, und dass in einem vollständig eingerasteten Zustand des Rastmechanismus (3) und einem nicht betätigten Zustand des Lösegetriebes der Schlauch (2) zwischen der Rippe (17) des Bügels (8) und dem Balken (16) durch die Kraft der von dem Rastmechanismus (3) gespannten Feder (7) so stark eingeklemmt ist, dass ein Durchfluss durch den Schlauch verhindert wird, und dass ausgehend von dem oben beschriebenen Zustand eine drückende Betätigung des Tasters (15) gegen die Kraft der Feder (7) den Balken (16) so weit von der Rippe (17) entfernt, dass der Verschluss des Schlauchs (2) aufgehoben wird, solange die Betätigung des Tasters (15) anhält.

2. Schlauchklemme nach Anspruch 1 **dadurch gekennzeichnet, dass** in der ersten Stufe der Betätigung der Rastmechanismus (3) durch einen ersten federnden Haken (4) gehalten wird und dass in der zweiten Stufe der Rastmechanismus durch einen oder mehrere weitere federnde Haken (5) gehalten wird, wobei der Bügel (8) die federnden Haken (4) und (5) führt, die sich in eine Traverse (18) einhaken, die Bestandteil des Gehäuses (9) ist.

3. Schlauchklemme nach Anspruch 2 **dadurch gekennzeichnet, dass** die erste und die zweite Stufe der Betätigung des Rastmechanismus (3) durch einen Druck auf den gelenkig an dem Gehäuse (9) abgestützten Bügel (8) bewirkt werden, unterschieden nur durch die Kraft oder den Weg der Betätigung.

4. Schlauchklemme nach Anspruch 1 **dadurch gekennzeichnet, dass** der Bügel (8) so weit von dem Gehäuse (9) abklappbar ist, dass der Schlauch (2) sich in gehäuseseitige halbrunde Aufnahmen (10) und in bügelseitige halbrunde Aufnahmen (11) einlegen lässt, womit die Schlauchklemme seitlich auf den Schlauch aufgesetzt wird.

5. Schlauchklemme nach einem der Ansprüche 1 bis 4 **dadurch gekennzeichnet, dass** der aus Kunststoff hergestellte Bügel (8) mittels eines Filmscharniers (12) aus Kunststoff mit dem ebenfalls aus Kunststoff hergestellten Gehäuse (9) verbunden ist.

6. Schlauchklemme nach einem der Ansprüche 1 bis 4 **dadurch gekennzeichnet, dass** der Bügel (8) mittels einer aus Haken an einem Bauteil und Ausnehmungen an dem anderen Bauteil gelenkig mit dem Gehäuse (9) verbunden ist.

7. Schlauchklemme nach Anspruch 1 **dadurch gekennzeichnet, dass** alle Bestandteile der Schlauchklemme aus dem gleichen Kunststoff mit einem gemeinsamen Werkzeug herstellbar sind und das Werkzeug keinen Schieber enthält, wobei die Schlauchklemme in einem weit geöffneten Zustand geformt wird und in diesem Zustand die Bauteile keine Hinterschnitte aufweisen.

## Claims

1. Hose clamp (1) for placing on a highly elastic hose (2), wherein the hose clamp (1) has a housing (9), a clip (8) and a latching mechanism (3) connecting the two components, said latching mechanism (3), after latching fully in place, keeping the hose clamp in a non-displaceable manner on the hose and also closing the hose,
wherein the hose clamp (1) contains a releasing mechanism (6) which, upon actuation, maintaining the latching function of the latching mechanism (3), allows the hose to spring back into an open form, for as long as the releasing mechanism continues to be actuated,
wherein the latching mechanism (3) is designed for two-stage actuation, and wherein the latching mechanism, after the hose clamp (1) has been placed on the hose (2) and said latching mechanism has been actuated in a first stage, keeps the hose clamp on the hose in a displaceable manner in receptacles (10) and (11), but does not yet close it,
and wherein the latching mechanism, after being actuated in a second stage, both keeps the hose clamp in a non-displaceable manner on the hose and also closes the hose in that it clamps it between a rib (17) and a bar (16) and in the process tensions a spring (7) supporting the bar,
**characterized in that** the releasing mechanism (6) consists of a button (15), the bar (16) and the spring (7), and **in that**, in a fully latched state of the latching mechanism (3) and a non-actuated state of the releasing mechanism, the hose (2) is clamped so strongly between the rib (17) of the clip (8) and the bar (16) by the force of the spring (7) tensioned by the latching mechanism (3) that throughflow through the hose is prevented, and **in that**, starting from the above-described state, pushing actuation of the button (15) counter to the force of the spring (7) moves the bar (16) away from the rib (17) to such an extent that the closure of the hose (2) is undone, for as long as the button (15) continues to be actuated.

2. Hose clamp according to Claim 1, **characterized in that**, in the first stage of actuation, the latching mechanism (3) is held by a first resilient hook (4), and **in that**, in the second stage, the latching mechanism is held by one or more further resilient hooks (5), wherein the clip (8) guides the resilient hooks (4) and (5), which hook into a cross member (18) which is a constituent part of the housing (9).

3. Hose clamp according to Claim 2, **characterized in that** the first and the second stage of actuation of the latching mechanism (3) are brought about by a pressure on the clip (8), supported in an articulated manner on the housing (9), said stages of actuation differing only by the force or the travel of the actuation.

4. Hose clamp according to Claim 1, **characterized in that** the clip (8) is able to be flapped away from the housing (9) to such an extent that the hose (2) can be laid in housing-side semi-circular receptacles (10) and in clip-side semi-circular receptacles (11), whereby the hose clamp is placed laterally on the hose.

5. Hose clamp according to one of Claims 1 to 4, **characterized in that** the clip (8), produced from plastics material, is connected to the housing (9), likewise produced from plastics material, by means of a film hinge (12) made of plastics material.

6. Hose clamp according to one of Claims 1 to 4, **characterized in that** the clip (8) is connected to the housing (9) in an articulated manner by means of a made up of hooks on one component and cutouts in the other component.

7. Hose clamp according to Claim 1, **characterized in that** all constituent parts of the hose clamp are producible from the same plastics material with a common tool, and the tool does not contain a slide, wherein the hose clamp is moulded in a wide-open state and, in this state, the components do not have any undercuts.

## Revendications

1. Bride de serrage pour tuyaux (1) à poser sur un tuyau hautement élastique (2), dans laquelle la bride de serrage pour tuyaux (1) présente un boîtier (9), un étrier (8) et un mécanisme d'encliquetage (3) reliant les deux composants, qui après l'encliquetage complet maintient la bride de serrage pour tuyaux de façon immobile sur le tuyau et ferme également le tuyau,
dans laquelle la bride de serrage pour tuyaux (1) contient un organe de libération (6) qui lors d'un actionnement, avec maintien de la fonction d'encliquetage du mécanisme d'encliquetage (3), permet un retour élastique du tuyau dans une forme ouverte, aussi longtemps que l'actionnement de l'organe de libération subsiste,
dans laquelle le mécanisme d'encliquetage (3) est conçu pour un actionnement en deux étapes, et dans laquelle le mécanisme d'encliquetage, après une pose de la bride de serrage pour tuyaux (1) sur le tuyau (2) et son actionnement dans une première étape, maintient la bride de serrage pour tuyaux de façon déplaçable dans des logements (10) et (11) sur le tuyau, mais ne le ferme pas encore,
et dans laquelle le mécanisme d'encliquetage, après son actionnement dans une deuxième étape, maintient la bride de serrage pour tuyaux de façon immobile sur le tuyau et ferme également le tuyau, du fait qu'il le serre entre une nervure (17) et une barre (16) et tend ainsi un ressort (7) soutenant la barre,
**caractérisée en ce que** l'organe de libération (6) se compose d'un bouton (15), de la barre (16) et du ressort (7), et **en ce que**, dans un état entièrement encliqueté du mécanisme d'encliquetage (3) et un état non actionné de l'organe de libération, le tuyau (2) est serré entre la nervure (17) de l'étrier (8) et la barre (16) par la force du ressort (7) tendu par le mécanisme d'encliquetage (3) à un point tel qu'un écoulement à travers le tuyau soit empêché, et **en ce qu'**en partant de l'état décrit ci-dessus un actionnement de pression du bouton (15) contre la force du ressort (7) éloigne la barre (16) de la nervure (17) de telle manière que la fermeture du tuyau (2) soit supprimée, aussi longtemps que l'actionnement du bouton (15) subsiste.

2. Bride de serrage pour tuyaux selon la revendication 1, **caractérisée en ce que** dans la première étape d'actionnement le mécanisme d'encliquetage (3) est maintenu par un premier crochet élastique (4) et **en ce que** dans la deuxième étape le mécanisme d'encliquetage est maintenu par un ou plusieurs autre(s) crochet(s) élastique(s) (5), dans laquelle l'étrier (8) guide les crochets élastiques (4) et (5), qui s'accrochent dans une traverse (18), qui est une partie constituante du boîtier (9) .

3. Bride de serrage pour tuyaux selon la revendication 2, **caractérisée en ce que** la première et la deuxième étapes d'actionnement du mécanisme d'encliquetage (3) sont provoquées par une pression sur l'étrier (8) appuyé de façon articulée sur le boîtier (9), et ne diffèrent que par la force ou la course de l'actionnement.

4. Bride de serrage pour tuyaux selon la revendication 1, **caractérisée en ce que** l'étrier (8) peut être rabattu à une distance telle du boîtier (9), que le tuyau (2) puisse être introduit dans des logements semi-circulaires côté boîtier (10) et dans des logements semi-circulaires côté étrier (11), la bride de serrage pour tuyaux étant ainsi posée latéralement sur le tuyau.

5. Bride de serrage pour tuyaux selon l'une quelconque des revendications 1 à 4, **caractérisée en ce que** l'étrier (8) fabriqué en matière plastique est relié au boîtier (9) également fabriqué en matière plastique au moyen d'une charnière pelliculaire (12) en matière plastique.

6. Bride de serrage pour tuyaux selon l'une quelconque des revendications 1 à 4, **caractérisée en ce que** l'étrier (8) est relié de façon articulée au boîtier (9) au moyen d'une composée de crochets sur un composant et d'évidements sur l'autre composant.

7. Bride de serrage pour tuyaux selon la revendication 1, **caractérisée en ce que** tous les composants de la bride de serrage pour tuyaux peuvent être fabriqués à partir de la même matière plastique avec un outil commun et l'outil ne comporte pas de coulisseau, dans laquelle la bride de serrage pour tuyaux est formée dans un état largement ouvert et dans cet état les composants ne présentent pas de contre-dépouilles.
